(19) Europäisches Patentamt
European Patent Office
Office européen des brevets

(11) **EP 4 152 336 A1**

(12) **EUROPEAN PATENT APPLICATION**

(43) Date of publication:
**22.03.2023 Bulletin 2023/12**

(21) Application number: **21306288.8**

(22) Date of filing: **17.09.2021**

(51) International Patent Classification (IPC):
**G16C 20/50** *(2019.01)*     **G16C 20/70** *(2019.01)*
**G06N 3/04** *(2006.01)*     **G06N 3/08** *(2006.01)*

(52) Cooperative Patent Classification (CPC):
**G06N 3/08; G06N 3/045; G16C 20/50; G16C 20/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TotalEnergies OneTech
92400 Courbevoie (FR)**

(72) Inventors:
• **ALAMI, Reda
  Massy, 91300 (FR)**
• **PEREIRA, Cécile
  Palaiseau, 91120 (FR)**
• **PANTANO, Diego
  Paris, 75014 (FR)**
• **ARAYA, Mauricio
  Houston, TX 77005 (US)**
• **AKHIYAROV, Denis
  Katy, TX 77005 (US)**
• **DAI, Zhenwei
  Houston, TX 77025 (US)**

(74) Representative: **Plasseraud IP
66, rue de la Chaussée d'Antin
75440 Paris Cedex 09 (FR)**

(54) **METHOD AND COMPUTING SYSTEM FOR MOLECULAR DESIGN VIA MULTI-TASK REINFORCEMENT LEARNING**

(57) The present disclosure relates to a computer implemented method (10) for molecular design via reinforcement learning, for designing molecules optimizing a predetermined criterion representative of at least one physicochemical property, wherein molecular design is performed by selecting a sequence of modifications to an initial molecule according to a selection policy, wherein the selection policy uses a Q-function which predicts a cumulative criterion score associated to subsequent molecules obtained by subsequent modifications, said Q-function being modeled by a trained neural network, said method (10) comprising:
- a pre-training phase (11) of a neural network modeling the Q-function, yielding a pre-trained neural network, wherein the pre-training phase comprises $N > 1$ reinforcement learning pre-training tasks applied to $N$ respective different training molecules,
- a training phase (12) of the pre-trained neural network using reinforcement learning, yielding the trained neural network, by applying different sequences of modifications to the initial molecule.

Fig. 2

## Description

### TECHNICAL FIELD

This disclosure relates to the field of molecular design and relates more particularly to molecular design using reinforcement learning.

### BACKGROUND ART

In chemistry, molecular design aims at finding new molecules with desirable physico-chemical properties.

Recently, machine learning has played an important role in molecular design. Compared to the traditional molecular design approaches, machine learning can significantly reduce the time and cost for identifying new molecules with good physico-chemical properties. Reinforcement learning is one the approaches considered for molecular design.

Reinforcement learning directly learns the actions that can generate molecules with desired physico-chemical properties, where the physico-chemical properties that need to be optimized are represented by a criterion to be optimized. In other words, the criterion (also known as reward in the context of reinforcement learning) is a function that depends on the physico-chemical properties to be optimized and that evaluates how good a molecule is with respect to these physico-chemical properties. Hence, molecular design with respect to these physico-chemical properties corresponds to searching for molecules that optimize this criterion (reward).

Reinforcement learning typically takes an initial molecule and learns the actions to modify the initial molecule into high quality molecules with respect to the criterion (reward). However, as a cost, training a reinforcement learning model is computationally expensive. It usually takes thousands of iterations to train a reinforcement learning model for molecular design.

Molecule deep Q-network, MolDQN, is a well-known reinforcement learning algorithm for molecular design [Zhou+19]. It formulates the modifications of an initial molecule as a Markov decision process. It provides a wide range of actions to modify the initial moelcule, such as removing bonds, adding bonds and adding atoms. As any reinforcement algorithm, the MolDQN algorithm considers a function, referred to as Q-function in the context of reinforcement learning, which predicts the future rewards when applying a given action (modification) in a given state (molecule). This Q-function is used for determining a selection policy, i.e. a policy used to select modifications to be applied to a given initial molecule in order to optimize future reward(s). This Q-function is not known *a priori* and is learned via reinforcement learning. The MolDQN algorithm includes a deep neural network, referred to as deep Q-network, in order to model the Q-function, and the reinforcement learning scheme aims at training this deep Q-network.

However, like for other reinforcement learning algorithms, training a MolDQN model is also time-consuming. For example, if willing to optimize the QED (quantitative estimation of drug-likeness) of a molecule (i.e. the criterion/reward is representative of the QED of the molecule), the MolDQN algorithm typically takes around four thousands iterations before the loss function converges, which typically takes several hours.

Another limitation of the MolDQN algorithm is that the trained deep Q-network is strongly dependent on the considered initial molecule. In other words, it cannot be applied as such to another initial molecule. Hence, if another initial molecule is considered, it is necessary to re-train from scratch another deep Q-network. Usually, in the context of molecular design, there might be hundreds or even thousands of candidate initial molecules (usually molecules that are already known to be good with respect to the considered physico-chemical properties), such that training a deep Q-network for each candidate initial molecule could take weeks, if not months.

### SUMMARY

The present disclosure aims at improving the situation. In particular, the present disclosure aims at overcoming at least some of the limitations of the prior art discussed above, by proposing a solution that can reduce the training time required for training the reinforcement learning model for a specific initial molecule, while still being able to identify good molecules, or that can find better molecules when the training time is constrained.

According to a first aspect, the present disclosure relates to a computer implemented method for molecular design via reinforcement learning, for designing molecules optimizing a predetermined criterion representative of at least one physico-chemical property, wherein molecular design is performed by applying a sequence of modifications to an initial molecule, wherein each modification applied to the initial molecule is selected in a set of possible modifications according to a selection policy, wherein the selection policy uses a function, referred to as Q-function, which predicts a cumulative criterion score associated to subsequent molecules obtained by subsequent modifications, wherein said Q-function is modeled by a trained neural network. Said molecular design method comprises:

- a pre-training phase of a neural network modeling the Q-function, yielding a pre-trained neural network, wherein the pre-training phase comprises $N > 1$ pre-training tasks applied to $N$ respective different training molecules, wherein each pre-training task uses reinforcement learning for training a local neural network modeling the Q-function by applying different sequences of modifications to the training molecule, wherein the pre-trained neural network is determined based on the $N$ local neural networks, and

- a training phase of the pre-trained neural network using reinforcement learning, yielding the trained neural network, by applying different sequences of modifications to the initial molecule.

Hence, as in the MolDQN algorithm, the present disclosure relies on reinforcement learning, wherein the Q-function is modeled by a neural network.

In the present disclosure, the training of the neural network comprises a pre-training phase, which uses multi-task reinforcement learning, and a training phase which uses reinforcement learning.

Multi-task reinforcement learning tries to learn a reinforcement learning model that can be generalized to multiple tasks. For molecular design, each task is associated to a different training molecule. Hence, in the present disclosure, the multi-task reinforcement learning trains the neural network with a plurality of different training molecules. This multi-task pre-training therefore generates more training examples and explores a larger region of the molecular space than the conventional MolDQN algorithm. Hence, the pre-trained neural network obtained is more general than the trained deep Q-network of the MolDQN algorithm in that it can be expected to scale better to different initial molecules.

Then the pre-trained neural network is trained via reinforcement learning for the considered initial molecule. Hence, the pre-trained neural network is fine tuned to better predict the Q-values of the Q-function when considering a specific initial molecule to be optimized. By fine tuning the pre-trained neural network with respect to the considered initial molecule, it is possible to identify modifications to the initial molecule that may further optimize the rewards.

At this stage, it is emphasized that the pre-training phase may be executed beforehand. The resulting pre-trained neural network, which may be used for a variety of different initial molecules, may therefore be computed once for all, stored in e.g. a database, and retrieved on the fly when needed to optimize a new initial molecule with respect to the same criterion. Since the pre-trained neural network can be computed once and stored beforehand, the time required to optimize a specific initial molecule corresponds to the time required to perform the training phase for the pre-trained neural network. Since the pre-trained neural network is not arbitrary, the number of iterations required to fine tune it with respect to the considered initial molecule can be lower than the number of iterations required to fully train the deep Q-network in the MolDQN algorithm with respect to the same initial molecule. Typically, while training the deep Q-network in the MolDQN algorithm may typically require 4000 iterations or more, comparable optimization results may be obtained by training the pre-trained neural network for around 1000 iterations. Hence, the training phase of the pre-trained neural network of the present disclosure can be accomplished much faster than the training phase in the MolDQN algorithm while yielding comparable optimization results.

The training molecules need not to include the molecule that will be used as initial molecule during molecular design. However, the training molecules are not chosen arbitrarily and are for instance molecules which are already considered to have good physico-chemical properties with respect to the same criterion to be optimized and/or molecules of a same type (e.g. petrochemical molecules).

In specific embodiments, the molecular design method can further comprise one or more of the following optional features, considered either alone or in any technically possible combination.

In specific embodiments, the pre-training phase comprises, for each pre-training task, iterating steps of:

- applying a sequence of modifications to the training molecule,
- determining criterion scores of molecules obtained by applying the sequence of modifications to the training molecule,
- determining updating parameters for the local neural network based on the determined criterion scores,

and the neural network is updated based on the updating parameters of the $N$ local neural networks, at each iteration. The training phase comprises iterating steps of:

- applying a sequence of modifications to the initial molecule,
- determining criterion scores of molecules obtained by applying said sequence of modifications to the initial molecule,
- updating the pre-trained neural network based on the determined criterion scores.

In specific embodiments, during the pre-training phase, updating the neural network at each iteration comprises:

- determining average updating parameters by averaging the updating parameters of the N local neural networks,
- updating the neural network based on the average updating parameters.

In specific embodiments, during the pre-training phase and the training phase, the criterion score of a molecule is determined by using a machine learning model previously determined by supervised learning and/or by performing a simulation to evaluate physico-chemical properties of said molecule.

In specific embodiments, during the pre-training phase and the training phase, the machine learning model is used to determine a criterion score by default and the simulation is used to determine a criterion score only when predetermined conditions are satisfied.

In specific embodiments, wherein the pre-training phase comprises a first number of iterations and the training phase comprises a second number of iterations, and the first number of iterations is strictly higher than the second number of iterations.

In specific embodiments, the pre-training phase and the training phase use a $\varepsilon$-greedy method for selecting a modification to a training molecule and to the initial molecule, wherein a modification is selected randomly with a probability $\varepsilon$ and is selected based on the Q-function with a probability $(1 - \varepsilon)$ and wherein, in both the pre-training phase and the training phase, the probability $\varepsilon$ decreases as the number of evaluated sequences of modifications increases.

In specific embodiments, the probability $\varepsilon$ is initialized at a value $\varepsilon_{PRE}$ for the pre-training phase and at a value $\varepsilon_{TR}$ for the training phase, and wherein the value $\varepsilon_{PRE}$ is strictly higher than the value $\varepsilon_{TR}$.

In specific embodiments, the number N of tasks/training molecules is higher or equal than 20, or higher or equal than 40.

In specific embodiments, the criterion includes an evaluation of a synthetic accessibility, SA, of the molecule.

In specific embodiments, the training molecules and the initial molecule are petrochemical molecules.

In specific embodiments, the petrochemical molecules considered are lubricant additives molecules and/or the criterion to be optimized is representative of at least one physico-chemical property chosen from: anti-wear, detergent, dispersant, biocide, friction modifiying, viscosity modifiying, antifoaming, corrosion inhibiting, electrical conductivity, thermic conductivity, ecotoxicity, heat capacity, kinematic viscosity measured at 100°C and antioxidant properties.

In specific embodiments, each sequence of modifications comprises a number $K > 1$ of modifications and the Q-function includes a weighting factor applied to each criterion score in the cumulative criterion score and said weighting factor increases with the rank of the modification in the sequence of modifications.

According to a second aspect, the present disclosure relates to a computer program product comprising instructions which, when executed by one or more processors, configure said one or more processors to carry out a molecular design method according to any one of the embodiments of the present disclosure.

According to a third aspect, the present disclosure relates to a computer-readable storage medium comprising instructions which, when executed by one or more processors, configure said one or more processors to carry out a molecular design method according to any one of the embodiments of the present disclosure.

According to a fourth aspect, the present disclosure relates to a computing system for molecular design via reinforcement learning, wherein said system comprises at least one memory and one or more processors configured to carry out a molecular design method according to any one of the embodiments of the present disclosure.

## BRIEF DESCRIPTION OF DRAWINGS

The invention will be better understood upon reading the following description, given as an example that is in no way limiting, and made in reference to the figures which show:

- Figure 1: a schematic represention of possible modifications that can be applied to molecules during reinforcement learning,
- Figure 2: a flow chart illustrating the main steps of an exemplary embodiment of a molecular design method,
- Figure 3: a flow chart illustrating the main steps of an exemplary embodiment of a pre-training phase,
- Figure 4: a flow chart illustrating the main steps of an exemplary embodiment of a training phase,
- Figure 5: curves representing experimental results on the convergence speed of three different molecular design algorithms,
- Figure 6: curves representing experimental results on the criterion scores obtained for three different molecular design algorithms,
- Figure 7: a schematic representation of an exemplary embodiment of a computing system for molecular design.

In these figures, references identical from one figure to another designate identical or analogous elements. For reasons of clarity, the elements shown are not to scale, unless explicitly stated otherwise.

## DESCRIPTION OF EMBODIMENTS

As indicated above, the present disclosure relates inter alia to a method and computing system for molecular design via reinforcement learning. The molecular design aims at finding good molecules with respect to a predetermined criterion. Basically, the criterion (also known as reward in the context of reinforcement learning) is a function that depends on a set of one or more physico-chemical properties to be optimized and that evaluates how good a given molecule is with respect to these physico-chemical properties.

As discussed above, the MolDQN algorithm proposed in [Zhou+19] is an example of reinforcement learning molecular design algorithm. In the sequel, we first introduce some notations in the context of the MolDQN algorithm, that will be used afterwards to describe the proposed solution.

The MolDQN algorithm uses a deep Q-network (DQN) model for molecular design. The model takes an input molecule (called initial molecule) and generates molecules satisfying some desired physico-chemical properties through multiple steps of modifications on the initial molecule. A molecule is represented by a SMILES string, and then converted to a numeric vector using the Morgan fingerprint as the input of the deep Q-network. The number of modification steps is limited up to $K$ steps (for instance $K = 20$). If we let $m_0$ be the initial molecule, then the MolDQN algorithm generates a final molecule through a modification path:

$$m_0 \xrightarrow{a_1} m_1 \xrightarrow{a_2} \cdots \xrightarrow{a_K} m_K$$

wherein $m_k$ is the molecule generated at step $k$, and $a_k$ is the action (also referred to as modification in the present disclosure) taken to modify molecule $m_k$. The goal of a MolDQN algorithm is to learn a modification path (a selection policy) to generate molecules which optimize a predetermined criterion. Here, the criterion (reward) is a function designed by users to measure the quality of the generated molecules. For example, if we want to generate molecules with large QED, the reward $r(m)$ (also referred to as criterion score in the present disclosure) of a molecule m is equal to the QED value of that molecule, i.e. $r(m) = QED(m)$.

The MolDQN algorithm uses a deep Q-network to model a function referred to as Q-function which predicts the future rewards (Q-value) of taking an action $a_k$ at step $k$. Mathematically, for a selection policy $\pi$ that takes a sequence of actions $a = (a_1, a_2, \cdots, a_K)$, the Q-value may for instance be expressed as:

$$Q(\pi, m_k) = \mathbb{E}_\pi(\sum_k^K \gamma^{K-k} r_k) \qquad (1)$$

wherein $\mathbb{E}_\pi$ denotes taking an expectation with respect to the selection policy $\pi$, $r_k$ is the criterion score (reward) at step $k$ and $0 < \gamma < 1$ is a discount factor. As can be seen in this expression, a weighting factor $\gamma^{K-k}$ is applied to the criterion score $r_k$. The weighting factor $\gamma^{K-k}$ increases with $k$ and reaches 1 when $k = K$. This may be used to focus on long term rewards rather than on short term rewards, if we want to search for final molecules (i.e. molecule $m_K$) with high criterion scores. Typically, the modifications (actions) can be selected in a predetermined set of possible modifications. For instance, if we have $W$ possible modifications $a_k^{(1)}, a_k^{(2)}, \ldots, a_k^{(W)}$ to modify $m_{k-1}$ to $m_k$, then the Q-function, modeled by the deep Q-network, predicts the Q-value of each possible modification $a_k^{(i)}$ and the selection policy $\pi$ typically consists in selecting the modification with the largest Q-value.

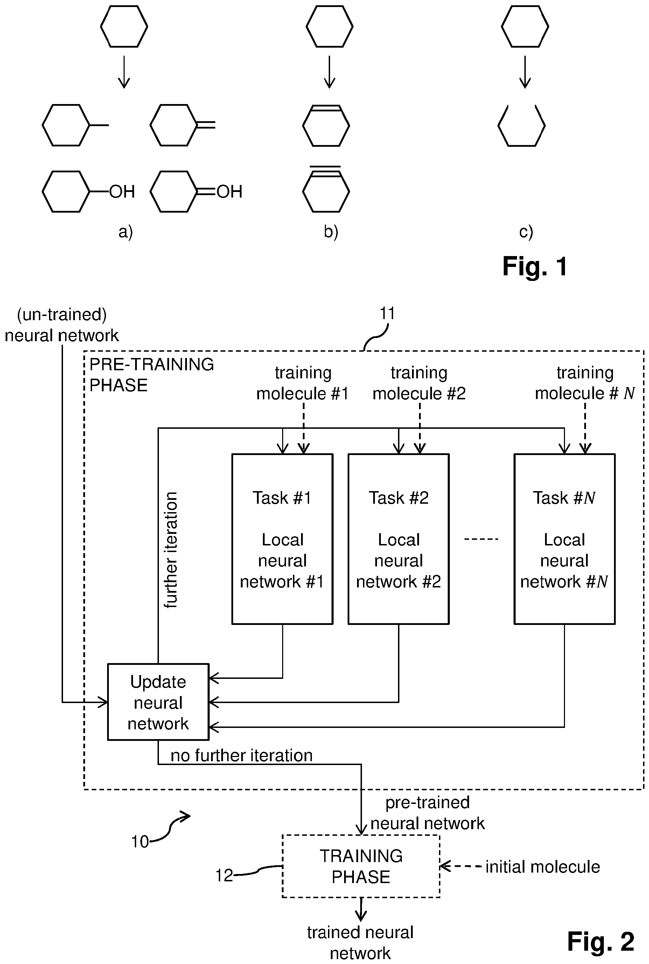

To implement the MolDQN algorithm, we need to specify the possible modifications allowed to modify the molecules. The MolDQN algorithm in [Zhou+19] provides three groups of modifications, including atom addition (part a) of figure 1), bond addition (part b) of figure 1) and bond removal (part c) of figure 1). It is also possible to make no modification since it might be possible in some cases to generate good molecules using less than $K$ steps. To ensure the modifications of the molecules are feasible, the MolDQN algorithm in [Zhou+19] also incorporates the chemistry domain knowledge. For example, the MolDQN algorithm does not allow a triple bond between oxygens for the bond addition modification. Other constraints may also be applied when selecting a modification to be applied to a molecule.

In the MolDQN algorithm, the deep Q-network is initialized at random. Hence, it cannot accurately predict the Q-value of a modification before it actually converges. Hence, fully relying on the deep Q-network may bias to the inferior modifications and cannot generate high-quality molecules. This issue is also known as exploitation / exploration dilemma. Hence, it is recommended, at least at the beginning of the training of the deep Q-network, to explore diverse modifications that many generate high rewards. The MolDQN algorithm uses the $\varepsilon$-greedy method to train the deep Q-network. Hence, when selecting a modification to be carried out at a given step, the MolDQN algorithm:

- selects randomly (with a uniform distribution) a modification in the set of possible modifications with probability $\varepsilon$;
- selects the modification with the largest associated Q-value (according to the deep Q-network) with probability $(1 - \varepsilon)$.

During the early stage of the training, the MolDQN algorithm uses a large probability $\varepsilon$ to explore different actions. As the training progresses, the MolDQN algorithm decreases the probability $\varepsilon$ since the deep Q-network can provide a more accurate prediction of the future rewards. Typically, the MolDQN algorithm will perform a large number of iterations (also referred to as episodes in the context of reinforcement learning) with a sequence of $K$ modifications selected at each iteration. For instance, the probability $\varepsilon_t$ at the iteration (episode) $t \geq 1$ may be set as:

$$\varepsilon_t = 1.0 \times \alpha^{t-1}$$

wherein $0 < \alpha < 1$ is the learning rate and can for instance be set to 0.999. The value of $\alpha$ affects the convergence rate. A large $\alpha$ can explore more modifications and generate better molecules but slows down the convergence. On the contrary, a smaller $\alpha$ encourages faster convergence but may lead to less optimal generated molecules.

Figure 2 represents schematically the main steps of a molecular design method 10 according to the present disclosure.

As the MolDQN algorithm, the molecular design method 10 uses reinforcement learning to optimize molecules with respect to a predetermined criterion (reward function) which is representative of one or more physico-chemical properties that need to be optimized.

Also, the optimization is performed by applying a finite-length sequence of $K > 1$ modifications to an initial molecule, wherein each modification applied to the initial molecule is selected in a predetermined set of possible modifications. For instance, the possible modifications may correspond to the possible modifications in the MolDQN algorithm, i.e. atom addition, bond addition and bond removal (see figure 1). In some cases, the selection may also be subject to further constraints. Hence, the optimization of the criterion is only carried out over the molecular space which can be reached by applying at most $K > 1$ modifications to the considered initial molecule.

Each modification applied within a sequence of modifications is selected according to a selection policy $\pi$, and the selection policy $\pi$ uses a Q-function which predicts cumulative criterion scores (rewards). For instance, the Q-function is expressed as indicated in equation (1) above, with or without the weighting factor. However, other expressions of the Q-function may also be considered in the present disclosure.

As in the MolDQN algorithm, each molecule may be represented by a SMILES string, and then converted to a numeric vector using the Morgan fingerprint. However, other representations of the molecules may also be considered. For instance, in other embodiments, the molecules may be represented by e.g. graphs.

As in the MolDQN algorithm, the molecular design method 10 uses a neural network, preferably a deep neural network. For instance, the neural network may comprise a four-layer fully connected network with hidden sizes of [1024, 512, 128, 32] and ReLu activations, as in the MolDQN algorithm in [Zhou+19]. However, other architectures may be considered for the neural network in the present disclosure.

As illustrated by figure 2, the molecular design method 10 comprises two main phases:

- a pre-training phase 11 which pre-trains the neural network and outputs a pre-trained neural network,
- a training phase 12 which takes the pre-trained neural network as input and trains it via reinforcement learning with respect to the considered initial molecule to optimize.

In the present disclosure, the pre-training phase 11 uses multi-task reinforcement learning applied to a plurality of different training molecules. The training phase 12 then performs a fine tuning of the pre-trained neural network with respect to the considered initial molecule, via reinforcement learning.

Multi-task reinforcement training is used to train a single model that can be extended to different tasks (see e.g. [Yu+19]). In the present disclosure, $N > 1$ reinforcement learning pre-training tasks are executed during the pre-training phase 11. Each of these N pre-training tasks trains a local (i.e. task specific) neural network (all $N$ local neural networks having the same structure) with respect to the same criterion but applied to different respective training molecules. Hence, these $N$ local neural networks model the same Q-function but explore different regions of the molecular space. For instance, the number $N$ of pre-training tasks/training molecules is higher or equal than 10, or higher or equal than 20, or even higher or equal than 40.

A neural network, having the same structure as the $N$ local neural networks, is trained during the pre-training phase 11 by using the $N$ local neural networks. To avoid confusion with the local neural networks, this neural network can be referred to as "global" in the sequel, in the description of the pre-training phase 11. The (global) neural network and the $N$ local neural networks are identical upon initialization. For instance, the parameters of the (global) neural network may be initialized randomly, and the same parameters are used to initialize the local parameters of the N local neural networks.

The pre-trained (global) neural network, which is the output of the pre-training phase 11, is determined based on said $N$ local neural networks. Basically, at each iteration, each pre-training task determines updating parameters for its local neural network, usually as a local gradient aiming at minimizing a predetermined loss function. At each iteration, the parameters of the (global) neural network are updated based on the updating parameters of the N local neural networks, and the updated parameters of the (global) neural network are broadcast to the different pre-training tasks where they replace the local parameters of each local neural network, and so on. When a stop criterion is satisfied, for instance when the number of iterations executed has reached a predetermined number $N_{PRE}$, the pre-training phase 11 ends and the pre-trained (global) neural network corresponds for instance to the latest updated (global) neural network.

An issue usually encountered in multi-task reinforcement learning is the interference between the multiple tasks. Since each task is executed independently and computes its own training data and loss function value, this leads to deviations between the local updating parameters, e.g. between the local gradients. Also, the local gradients from different tasks may have conflicts (e.g. have negative cosine similarity). Yu et al. [Yu+19] try to deconflict the local gradients by removing the conflicting parts of the local gradients. If a local gradient is in conflict with other local gradients, the conflicting local gradient can be projected along a normal plane of the other local gradients to remove the conflicting part of that local gradient. In the molecular design context (in particular with the same criterion used in all pre-training $N$ tasks and a single Q-function), the inventors have found that the local gradients can be directly combined to update the (global) neural network while still achieving good performance on all the pre-training tasks. In particular, the updating parameters (i.e. local gradients) can be simply averaged to produce average updating parameters, and the (global) neural network can be updated by using directly the average updating parameters.

Of course, other methods can be used for updating the (global) neural network based on the updating parameters of all $N$ local neural networks, including the method described in [Yu+19]. However, the inventors have found that, in the present context of molecular design, the approach consisting in averaging the updating parameters achieved similar performance as the method described in [Yu+19], with lower computational complexity.

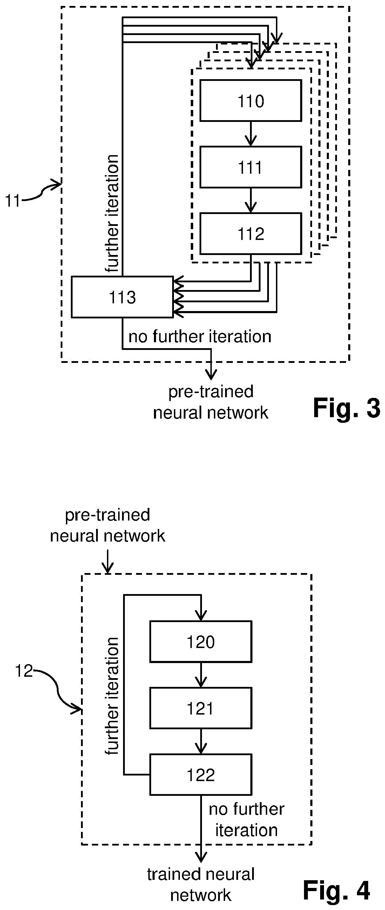

Figure 3 represents schematically the main steps of a preferred embodiment of the pre-training phase 11. As illustrated by figure 3, each task of the pre-training phase 11 comprises, at each iteration $t \geq 1$:

- a step 110 of applying a sequence of modifications to the training molecule,
- a step 111 of determining criterion scores of molecules obtained by applying the sequence of modifications to the training molecule,
- a step 112 of determining updating parameters for the local neural network based on the determined criterion scores.

During the step 110, the modifications are selected in the predetermined set of possible modifications. In preferred embodiments, the selection of the modifications may use a $\varepsilon$-greedy method. Hence, a modification is selected randomly with a probability $\varepsilon$ and is selected by maximizing the Q-function with a probability $(1 - \varepsilon)$. Preferably, the probability $\varepsilon$ decreases as the number of evaluated sequences of modifications increases, i.e. it decreases with t. For instance, a probability $\varepsilon_t = \varepsilon_{PRE} \times \alpha^{t-1}$ is used at iteration t, with $0 < \alpha < 1$.

During step 111, a criterion score (reward) is determined for each molecule obtained via the successive modifications of the sequence of modifications. The criterion is basically a function representative of one or more predetermined physical properties (examples are provided hereinbelow). In some cases, there might exist an analytical function for the criterion, in which case determining the criterion score consists in computing the value of the analytical function for the considered molecule. According to another example, it is possible to rely on machine learning for determining a criterion score of a given molecule. For instance, if the criterion scores (or the values of the considered physico-chemical properties) are known for a plurality of molecules, it is possible to determine beforehand a machine learning model of the criterion function via supervised learning. Then this machine learning model can be used during step 111 to determine the criterion score of the considered molecule. According to another example, it is possible to evaluate the physico-chemical properties of a given molecule by simulation, for instance DFT (density-functional theory) simulation, and to use the estimated physico-chemical properties to compute the criterion score. However, determing the criterion score via simulation might be time consuming, especially compared to a (previously trained) machine learning model. Hence, determing the criterion score via simulation is preferably limited to specific cases, e.g. when predetermined conditions are satisfied. For instance, the criterion score may be determined by simulation if e.g. the accuracy of the machine learning model for this molecule is expected to be low (for instance if the considered molecule is substantially different from those molecules used in the training set of the machine learning model). In such a case, the criterion score determined via simulation, and the associated molecule, can be advantageously added to the training set of the machine learning model of the criterion function.

During step 112, updating parameters are determined for the local neural network based on the determined criterion scores. These determined criterion scores can be used e.g. to compute Q-values, which can be used in a conventional manner in a predetermined loss function, and the updating parameters for the local neural network (typically the local gradients for the local neural network) aim at modifying the local neural network in order to minimize the loss function.

The steps 110, 111, 112 are executed at each iteration $t$ and for each of the $N$ pre-training tasks. At each iteration *t,* the (global) neural network is updated during a step 113 by using the updating parameters determined for all *N* local neural networks, for instance by averaging said updating parameters. The updated (global) neural network is then used to update all *N* local neural networks. If the stop criterion is not satisfied, the pre-training phase 11 proceeds with iteration *t* + 1. If the stop criterion is satisfied, the pre-trained neural network is obtained as the latest updated (global) neural network.

It is emphasized that the order of the steps, as illustrated in figure 3, is merely illustrative and in no way limiting. For instance, it is possible to select a modification and to determine its criterion score before selecting the subsequent modification, etc.

Figure 4 represents schematically the main steps of a preferred embodiment of the training phase 12. As illustrated by figure 4, the training phase 12 comprises, at each iteration $t' \geq 1$:

- a step 120 of applying a sequence of modifications to the initial molecule,
- a step 121 of determining criterion scores of molecules obtained by applying said sequence of modifications to the initial molecule,
- a step 122 of updating the pre-trained neural network based on the determined criterion scores.

Basically, the steps 120 and 121 are similar to the steps 110 and 111 of the pre-training phase 11, except that they are applied to the initial molecule and that they use the pre-trained neural network. Similarly, an $\varepsilon$-greedy method may be used during step 120, by using a probability $\varepsilon'$. Preferably, the probability $\varepsilon'$ decreases as the number of evaluated sequences of modifications increases, i.e. it decreases with *t'*. For instance, a probability $\varepsilon'_{t'} = \varepsilon_{TR} \times \alpha'^{t'-1}$ is used at iteration *t',* with $0 < \alpha' < 1$.

During step 122, updating parameters (e.g. gradients) can be determined as in step 112 of the pre-training phase 11, based on the determined criterion scores, and these updating parameters are used to update the parameters of the pre-trained neural network.

The steps 120, 121, 122 are executed at each iteration *t',* until a predetermined stop criterion is satisfied. For instance, the stop criterion is satisfied when the number of iterations executed has reached a predetermined number $N_{TR}$. If the stop criterion is not satisfied, the training phase 12 proceeds with iteration *t'* + 1. If the stop criterion is satisfied, the trained neural network is obtained as the latest updated pre-trained neural network.

It is emphasized that the order of the steps, as illustrated in figure 4, is merely illustrative and in no way limiting. For instance, it is possible to select a modification and to determine its criterion score before selecting the subsequent modification, etc.

As discussed above, the pre-trained neural network can be determined beforehand, once for all. Hence, when a new initial molecule is to be optimized, the pre-trained neural network can be retrieved on the fly from e.g. a database and used to carry out the training phase 12 for this initial molecule.

Thanks to the pre-training phase 11, the pre-trained neural network has already been able to predict the Q-values for many (training) molecules. Hence, we do not need to explore as much as in the pre-training phase 11. Accordingly, in specific embodiments, the probability $\varepsilon_{PRE}$ for the pre-training phase is strictly higher than the probability $\varepsilon_{TR}$. Preferably, $\varepsilon_{PRE} > 0.8$ (for instance $\varepsilon_{PRE} = 1.0$) and $\varepsilon_{TR} < 0.2$ (for instance, $\varepsilon_{TR} = 0.1$). The learning rates $\alpha$ and $\alpha'$ may be the same, for instance $\alpha = \alpha' = 0.999$.

Also, thanks to the pre-training phase 11, the training phase 12 can comprise fewer iterations than the pre-training phase 11, i.e. $N_{PRE} > N_{TR}$. Preferably, $N_{PRE} \geq 2 \times N_{TR}$, or $N_{PRE} \geq 4 \times N_{TR}$. For instance, the number $N_{PRE}$ of iterations of the pre-training phase 11 may be equal to 10000, and the number $N_{TR}$ of iterations of the training phase 12 may be equal to 1000. More importantly, since the pre-trained neural network has already been able to predict the Q-values for many (training) molecules, the number $N_{TR}$ of iterations of the training phase 12 may be lower, or even significantly lower than the number of iterations required for the MolDQN algorithm in [Zhou+19] to converge. Hence the training phase 12 of the present disclosure converges much faster than the training phase of the MolDQN algorithm in [Zhou+19], such that good molecules can be identified much faster, with similar results (as discussed hereinbelow). Also, in practical situations where the time available for designing new molecules is limited, the molecular design method 10 of the present disclosure will be able to span a broader region of the molecular space, to process more candidate initial molecules and, *in fine,* to identify better molecules than the conventional MolDQN algorithm. In other words, the molecular design method 10 of the present disclosure may find better molecules than the MolDQN algorithm when considering the same duration for the training phase 12 of the molecular design method 10 and the training phase of the MolDQN algorithm.

As discussed above, the optimization is carried out with respect to a predetermined criterion (reward function) which evaluates how good a given molecule is with respect to predetermined physico-chemical properties.

For instance, the molecules considered may correspond to drugs, as in [Zhou+19]. In such a case, the one or more physico-chemical properties evaluated may include for instance the QED (quantitative estimation of drug likeliness) score, the SA (synthetic accessibility) score (a large SA score means that the molecule is difficult to synthetize), etc.

For instance, the criterion score (reward) of a molecule m may be computed as follows:

$$r(m) = \text{QED}(m) - \lambda \times SA(m) \qquad (2)$$

wherein $\lambda$ is a regularization factor (for instance, $\lambda = 0.2$).

In preferred embodiments, the molecules considered are petrochemical molecules. In such a case, the one or more physico-chemical properties evaluated may include at least one physico-chemical property chosen in the following set of physico-chemical properties: anti-wear, detergent, dispersant, biocide, friction modifiying, viscosity modifiying, antifoaming, corrosion inhibiting, electrical conductivity (TE), thermic conductivity (TC), ecotoxity/toxicity, heat capacity, kinematic viscosity measured at 100°C and antioxidant properties. Preferably, the petrochemical molecules considered are lubricant additives molecules and the criterion to be optimized is representative of at least one physico-chemical property selected in the following set: anti-wear, detergent, dispersant, biocide, friction modifiying, viscosity modifiying, antifoaming, corrosion inhibiting, electrical conductivity (TE), thermic conductivity (TC), ecotoxity/toxicity, heat capacity, kinematic viscosity measured at 100°C and antioxidant properties. For instance, the criterion score (reward) of a molecule m may be computed as follows:

$$r(m) = \delta \times \text{TC}(m) + \beta \times \big(1 - \text{TE}(m)\big) - \lambda \times SA(m) \qquad (3)$$

herein $\lambda$, $\delta$ and $\beta$ are regularization factors (for instance, $\lambda = 0.2$). Such a criterion as expressed in equation (3) may for instance be used for evaluating molecules used in fluids for electric vehicles.

We now provide experimental results which compare the results obtained with the MolDQN algorithm in [Zhou+19] with the results obtained with the molecular design method 10 (referred to as "MT-MolDQN" algorithm in the sequel). We also compare with the results obtained with an algorithm implementing a pre-training phase and a training phase as in the molecular design method 10, in which the pre-training phase has a single task with a single training molecule (referred to as "PT-MolDQN" in the sequel).

In order to compare the results of the MT-MolDQN, MolDQN and PT-MolDQN algorithms, we evaluate their performance on generating high QED score molecules.

QED is widely used to measure the drug likeness of a molecule and ranges from 0 to 1. To search for molecules having better synthesizability, the criterion penalizes the large SA scores. In this experiment, the criterion score was computed according to the equation (2), with $\lambda = 0.2$.

The molecules in the ChEMBL dataset are used in this experiment. However, the original ChEMBL dataset is too large (it has millions of molecules) and using a few molecules for multi-task pre-training is not enough to explore such a huge dataset. Moreover, it is also usually difficult to optimize the QED score of molecules having a large weight. Therefore, we choose to select small weight molecules for our experiment. We pick the molecules with weight between 100 and 199 g/mol and containing aromatic rings from the ChEMBL dataset. After selection, there are 14799 molecules with legitimate SMILES strings, and we denote it as "ChEMBL small molecule dataset". To pre-train the neural network of the MT-MolDQN algorithm, we randomly select $N = 25$ training molecules from the ChEMBL small molecule dataset. The number $N_{PRE}$ of iterations of the pre-training phase 11 is set to 10000, for both the MT-MolDQN and PT-MolDQN algorithms. To evaluate the performance of the MT-MolDQN algorithm, we select 6 different initial molecules to be optimized from the ChEMBL small molecule dataset. The 6 initial molecules are split into two different groups, respectively small distance group and large distance group. The initial molecules in the small distance group have smaller Tanimoto distance to the training molecules used during the pre-training phase 11 of the MT-MolDQN algorithm. Three out of the six initial molecules are in the small distance group, and their SMILES strings are:

- Molecule A: "O-C(O)[C@H]1NCc2ccccc21" (mean Tanimoto distance with the 25 training molecules: 0.555),
- Molecule B: "Cc1ccc2c(c1)CNCC2" (mean Tanimoto distance with the 25 training molecules: 0.627),
- Molecule C: "CN[C@@H]1CCCc2ccccc21" (mean Tanimoto distance with the 25 training molecules: 0.556).

The other three initial molecules are in the large distance group, and their SMILES strings are:

- Molecule D: "COc1ccc(C23CNCC2C3)cc1" (mean Tanimoto distance with the 25 training molecules: 0.734),
- Molecule E: "CNC1(/C=C/c2cccnc2)CC1" (mean Tanimoto distance with the 25 training molecules: 0.876),
- Molecule F: "NCc1cccc(C(F)(F)F)c1" (mean Tanimoto distance with the 25 training molecules: 0.830).

For all algorithms, we set $\alpha = 0.999$. For the MolDQN algorithm, we set $\varepsilon = 1.0$. For the MT-MolDQN and PT-MolDQN algorithms, we set $\varepsilon_{PRE} = 1.0$ for the pre-training phase and $\varepsilon_{TR} = 0.1$ for the training phase.

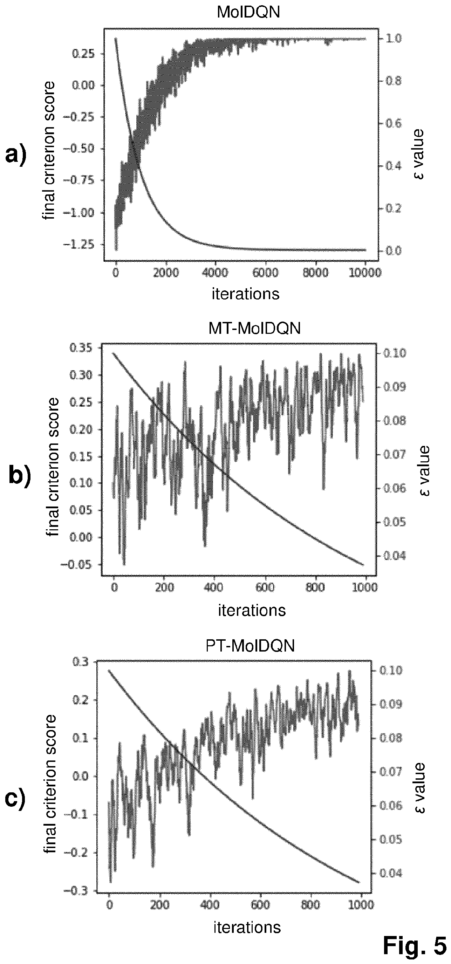

Figure 5 represents the convergence curves on the training phases on molecule A. More specifically, part a) of figure 5 shows the results for the MolDQN algorithm, part b) shows the results for the MT-MolDQN algorithm and part c) shows the results for the PT-MolDQN algorithm.

Compared to the MolDQN algorithm, the initial (first iterations) criterion scores (rewards) of the MT-MolDQN algorithm are much higher, which is reasonable since the MT-MolDQN algorithm is initialized with a pre-trained neural network. To achieve an average criterion score higher than 0.3, the training phase 12 of the MT-MolDQN algorithm takes only around 500 iterations while the MolDQN algorithm takes over 3000 iterations. Moreover, we can see that, compared to the PT-MolDQN algorithm, the MT-MolDQN algorithm also converges to a larger criterion score. Also, as can be seen in figure 5, the criterion scores at around 1000 iterations are much better for the MT-MolDQN algorithm than for the MolDQN algorithm.

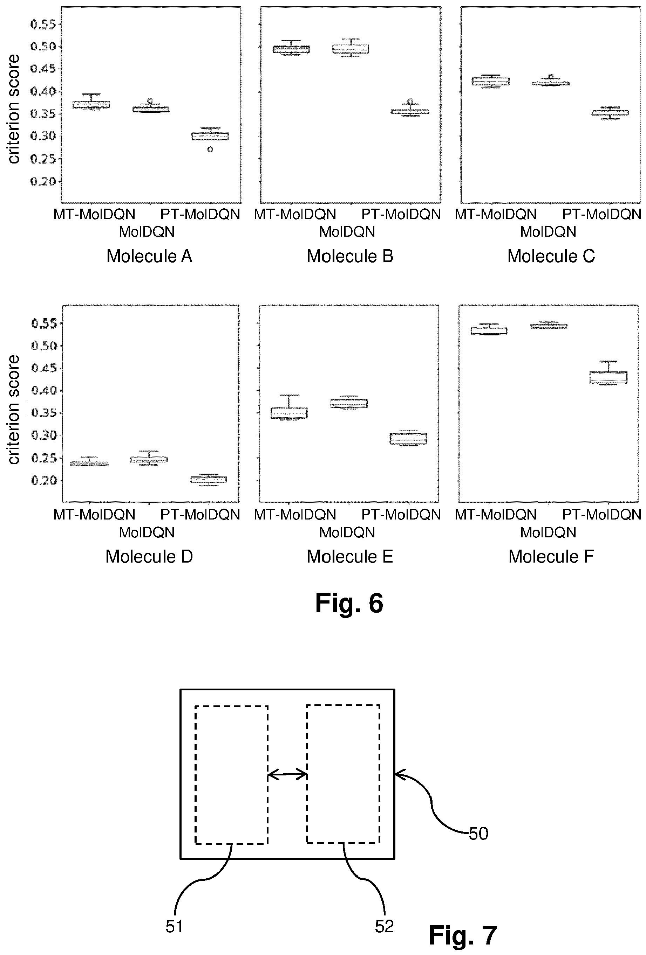

Figure 6 represents the criterion scores of the 10 best molecules generated by the three algorithms, for molecules A-F. In figure 6, the MolDQN algorithm has been trained over 4000 iterations, while the number $N_{TR}$ of iterations of the training phase 12 has been set to 1000 for the MT-MolDQN and PT-MolDQN algorithms.

As can be seen in figure 6, the MT-MolDQN algorithm achieves similar or even larger criterion scores compared to the MolDQN algorithm (with significantly fewer iterations), and achieves much better criterion scores compared to the PT-MolDQN algorithm. We also evaluate the performance of the MT-MolDQN algorithm on the 3 initial molecules from the large distance group. Although the 3 initial molecules vary a lot compared to the training molecules used during the multi-task pre-training phase 11, the performance of the MT-MolDQN algorithm is still comparable to the performance of the MolDQN algorithm. The criterion scores for the 10 best molecules are slightly worse than for the MolDQN algorithm when optimizing the molecules D and F, but the difference is not significant. Therefore, the distance between the initial molecule and the training molecules does not significantly affect the performance of the MT-MolDQN algorithm, suggesting the robustness of the MT-MolDQN algorithm in optimizing the QED scores.

Figure 7 represents an exemplary embodiment of a computing system 50 for molecular design via reinforcement learning. For example, the computing system 50 may comprise one or more processors 51 (which may colocated or distributed, e.g. in a cloud or high performance computing, HPC, architecture) and at least one memory 52 (magnetic hard disk, solid-state disk, optical disk, electronic memory, or any type of computer-readable storage medium) in which a computer program product is stored, in the form of a set of program-code instructions to be executed by the one or more processors in order to implement all or part of the steps of the molecular design method 10 according to the present disclosure. For instance, the pre-trained neural network(s), once obtained via a pre-training phase 11, can be stored in the at least one memory 52 of the computing system 50 for future use.

It is emphasized that the present invention is not limited to the above exemplary embodiments. Variants of the above exemplary embodiments are also within the scope of the present invention.

## REFERENCES

[Zhou+19] Z. Zhou, S. Kearnes, L. Li, R. N. Zare, and P. Riley: "Optimization of Molecules via Deep Reinforcement Learning", Scientific Reports 9.1 (2019), pages 1-10, https://doi.org/10.1038/s41598-019-47148-x

[Yu+19] T. Yu, S. Kumar, A. Gupta, S. Levine, K. Hausman, and C. Finn: "Multi-Task Reinforcement Learning without Interference", 2019

**Claims**

1. Computer implemented method (10) for molecular design via reinforcement learning, for designing molecules optimizing a predetermined criterion representative of at least one physico-chemical property, wherein molecular design is performed by applying a sequence of modifications to an initial molecule, wherein each modification applied to the initial molecule is selected in a set of possible modifications according to a selection policy, wherein the selection policy uses a function, referred to as Q-function, which predicts a cumulative criterion score associated to subsequent molecules obtained by subsequent modifications, wherein said Q-function is modeled by a trained neural network, said method being **characterized in that** it comprises:

   - a pre-training phase (11) of a neural network modeling the Q-function, yielding a pre-trained neural network, wherein the pre-training phase comprises $N > 1$ pre-training tasks applied to $N$ respective different training molecules, wherein each pre-training task uses reinforcement learning for training a local neural network modeling the Q-function by applying different sequences of modifications to the training molecule, wherein the pre-trained neural network is determined based on the $N$ local neural networks,

- a training phase (12) of the pre-trained neural network using reinforcement learning, yielding the trained neural network, by applying different sequences of modifications to the initial molecule.

2. Method (10) according to claim 1, wherein:

- the pre-training phase (11) comprises, for each pre-training task, iterating steps of:

   ◦ (110) applying a sequence of modifications to the training molecule,
   ◦ (111) determining criterion scores of molecules obtained by applying the sequence of modifications to the training molecule,
   ◦ (112) determining updating parameters for the local neural network based on the determined criterion scores,
   wherein the neural network is updated (113) based on the updating parameters of the $N$ local neural networks,

- the training phase (12) comprises iterating steps of:

   ◦ (120) applying a sequence of modifications to the initial molecule,
   ◦ (121) determining criterion scores of molecules obtained by applying said sequence of modifications to the initial molecule,
   ◦ (122) updating the pre-trained neural network based on the determined criterion scores.

3. Method (10) according to claim 2, wherein, during the pre-training phase (11), updating the neural network at each iteration comprises:

   - determining average updating parameters by averaging the updating parameters of the $N$ local neural networks,
   - updating the neural network based on the average updating parameters.

4. Method (10) according to any one of claims 2 to 3, wherein, during the pre-training phase (11) and the training phase (12), the criterion score of a molecule is determined by using a machine learning model previously determined by supervised learning and/or by performing a simulation to evaluate physico-chemical properties of said molecule.

5. Method (10) according to any one of claims 2 to 4, wherein the pre-training phase (11) comprises a first number of iterations and the training phase (12) comprises a second number of iterations, and the first number of iterations is strictly higher than the second number of iterations.

6. Method (10) according to any one of the preceding claims, wherein the pre-training phase (11) and the training phase (12) use a $\varepsilon$-greedy method for selecting a modification to a training molecule and to the initial molecule, wherein a modification is selected randomly with a probability $\varepsilon$ and is selected based on the Q-function with a probability $(1 - \varepsilon)$ and wherein, in both the pre-training phase and the training phase, the probability $\varepsilon$ decreases as the number of evaluated sequences of modifications increases.

7. Method (10) according to claim 6, wherein the probability $\varepsilon$ is initialized at a value $\varepsilon_{PRE}$ for the pre-training phase and at a value $\varepsilon_{TR}$ for the training phase, and wherein the value $\varepsilon_{PRE}$ is strictly higher than the value $\varepsilon_{TR}$.

8. Method (10) according to any one of the preceding claims, wherein $N$ is higher or equal than 20, or higher or equal than 40.

9. Method (10) according to any one of the preceding claims, wherein the criterion includes an evaluation of a synthetic accessibility, SA, of the molecule.

10. Method (10) according to any one of the preceding claims, wherein the training molecules and the initial molecule are petrochemical molecules.

11. Method (10) according to claim 10, wherein the petrochemical molecules considered are lubricant additives molecules and/or the criterion to be optimized is representative of at least one physico-chemical property chosen from: anti-wear, detergent, dispersant, biocide, friction modifiying, viscosity modifiying, antifoaming, corrosion inhibiting, electrical conductivity, thermic conductivity, ecotoxicity, heat capacity, kinematic viscosity measured at 100°C and antioxidant properties.

12. Method (10) according to any one of the preceding claims, wherein each sequence of modifications comprises a number $K$ of modifications and the Q-function includes a weighting factor applied to each criterion score in the cumulative criterion score, wherein said weighting factor increases with the rank of the modification in the sequence of modifications.

13. Computer program product comprising code instructions which, when executed by a computing system (50) comprising one or more processors (51), cause said one or more processors to carry out a molecular design method as claimed in any one of claims 1 to 12.

14. Computer-readable storage medium (52) comprising instructions which, when executed by a computing system (50) comprising one or more processors (51), cause said one or more processors to carry out a molecular design method as claimed in any one of claims 1 to 12.

15. Computing system (50) for molecular design via reinforcement learning, wherein said computing system (50) comprises at least one memory (52) and one or more processors (51) configured to carry out a molecular design method (10) as claimed in any one of claims 1 to 12.

**Fig. 1**

**Fig. 2**

11

further iteration

| 110 |

↓

| 111 |

↓

| 112 |

| 113 |

no further iteration

↓

pre-trained
neural network

**Fig. 3**

pre-trained
neural network

↓

12

further iteration

| 120 |

↓

| 121 |

↓

| 122 |

no further
iteration

↓

trained neural
network

**Fig. 4**

Fig. 5

Fig. 6

Fig. 7

Europäisches
Patentamt

European
Patent Office

Office européen
des brevets

# EUROPEAN SEARCH REPORT

Application Number

EP 21 30 6288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | ZHOU ZHENPENG ET AL: "Optimization of Molecules via Deep Reinforcement Learning", SCIENTIFIC REPORTS, vol. 9, no. 1, 24 July 2019 (2019-07-24), XP055895297, DOI: 10.1038/s41598-019-47148-x Retrieved from the Internet: URL:https://www.nature.com/articles/s41598-019-47148-x.pdf> * the whole document * | 1-15 | INV.<br>G16C20/50<br>G16C20/70<br>G06N3/04<br>G06N3/08 |
| A | DANIEL C ELTON ET AL: "Deep learning for molecular generation and optimization – a review of the state of the art", ARXIV.ORG, CORNELL UNIVERSITY LIBRARY, 201 OLIN LIBRARY CORNELL UNIVERSITY ITHACA, NY 14853, 11 March 2019 (2019-03-11), XP081270483, * the whole document * | 1-15 | |
| A | ZHANG CHENRUI ET AL: "Molecular Graph Generation with Deep Reinforced Multitask Network and Adversarial Imitation Learning", 2019 IEEE INTERNATIONAL CONFERENCE ON BIOINFORMATICS AND BIOMEDICINE (BIBM), IEEE, 18 November 2019 (2019-11-18), pages 326-329, XP033704107, DOI: 10.1109/BIBM47256.2019.8983277 [retrieved on 2020-02-04] * the whole document * | 1-15 | **TECHNICAL FIELDS SEARCHED (IPC)**<br>G16C<br>G06N |

-/--

The present search report has been drawn up for all claims

| Place of search | Date of completion of the search | Examiner |
|---|---|---|
| The Hague | 25 February 2022 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| Europäisches Patentamt / European Patent Office / Office européen des brevets | **EUROPEAN SEARCH REPORT** | Application Number |
| --- | --- | --- |
| | | EP 21 30 6288 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | NELSON VITHAYATHIL VARGHESE ET AL: "A Survey of Multi-Task Deep Reinforcement Learning", ELECTRONICS, vol. 9, no. 9, 22 August 2020 (2020-08-22), pages 1363-21, XP055756695, Basel, Switzerland ISSN: 2079-9292, DOI: 10.3390/electronics9091363 * the whole document * | 1-15 | |
| A,D | YU TIANHE ET AL: "Multi-Task Reinforcement Learning without Interference", NEURIPS 2019 OPTIMIZATION FOUNDATIONS OF REINFORCEMENT LEARNING WORKSHOP, 23 November 2019 (2019-11-23), pages 1-9, XP055895289, Retrieved from the Internet: URL:https://github.com/OptRL2019/optrl2019.github.io/raw/master/assets/accepted_papers/16.pdf> * the whole document * | 1-15 | |
| | | | **TECHNICAL FIELDS SEARCHED (IPC)** |

The present search report has been drawn up for all claims

| Place of search | Date of completion of the search | Examiner |
| --- | --- | --- |
| The Hague | 25 February 2022 | Denoual, Matthieu |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Z. ZHOU ; S. KEARNES ; L. LI ; R. N. ZARE ; P. RILEY.** Optimization of Molecules via Deep Reinforcement Learning. *Scientific Reports,* 2019, vol. 9 (1), 1-10

- **T. YU ; S. KUMAR ; A. GUPTA ; S. LEVINE ; K. HAUSMAN ; C. FINN.** *Multi-Task Reinforcement Learning without Interference,* 2019